Europäisches Patentamt

European Patent Office

Office européen des brevets

(19)

(11) **EP 1 366 709 A1**

(12) **EUROPEAN PATENT APPLICATION**

(43) Date of publication:
**03.12.2003 Bulletin 2003/49**

(51) Int Cl.⁷: **A61B 5/0285**

(21) Application number: **03006242.6**

(22) Date of filing: **20.03.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **15.05.2002 JP 2002140396**

(71) Applicant: **Colin Corporation
Komaki-shi, Aichi-ken (JP)**

(72) Inventors:
• **Ikenoue, Tsuyomu, Miyazaki Medical College
Miyazaki-gun, Miyazaki-ken (JP)**

• **Samejima, Hiroshi, Miyazaki Medical College
Miyazaki-gun, Miyazaki-ken (JP)**
• **Ito, Hisashi
Komaki-shi, Aichi-ken (JP)**

(74) Representative:
**Winter, Brandl, Fürniss, Hübner, Röss, Kaiser,
Polte Partnerschaft
Patent- und Rechtsanwaltskanzlei
Alois-Steinecker-Strasse 22
85354 Freising (DE)**

(54) **Fetal-pulse-wave-velocity-related-information obtaining apparatus**

(57) A fetal-pulse-wave-velocity-related-information obtaining apparatus (10), comprising a heartbeat-synchronous-signal detecting device (32) at least a portion of which is adapted to be worn on at least one of a fetus and a mother so as to detect a heartbeat-synchronous signal produced from a proximal portion of the fetus; a pulse-wave detecting device (42) which detects a pulse wave from a distal portion of the fetus; and a pulse-wave-velocity-related-information obtaining means (58) for iteratively obtaining, based on said detected signals, respective sets of pulse-wave-velocity-related information.

FIG. 1

EP 1 366 709 A1

## Description

BACKGROUND OF THE INVENTION

Field of the Invention

The present invention relates to a fetal-pulse-wave-velocity-related-information obtaining apparatus which obtains pulse-wave-velocity-related information from a fetus, and a childbirth monitoring apparatus having the function of obtaining pulse-wave-velocity-related information from a fetus. Here, pulse-wave-velocity-related information is defined as information that is related to a velocity at which a pulse wave propagates in a living subject; such as a pulse-wave velocity itself or a pulse-wave propagation time.

Related Art Statement

When a mother delivers an infant, there is a risk of fetal distress if blood pressure of the fetus is excessively low after uterine contraction (i.e., labor pain). Thus, a childbirth monitoring apparatus commonly has the function of monitoring degree of labor pain (i.e., degree of uterine contraction) of the mother and heart rate of the fetus.

A fetus is supplied through umbilical cord with blood and oxygen. However, during uterine contraction, blood cannot flow from the umbilical cord to the fetus. Thus, the fetus is periodically subjected to state of low oxygen. If the state of low oxygen lasts for a long time, the fetus is subjected to a risk of cerebral hypoxia. In addition, past studies have elucidated that before a fetus gets the risk of cerebral hypoxia, blood pressure of the fetus significantly decreases. However, the conventional childbirth monitoring apparatus cannot monitor the blood pressure of the fetus, and accordingly a medical person recognizes the risk of cerebral hypoxia of the fetus based on only change of labor-pain waveform and change of heart rate of the fetus. Thus, it has been difficult to quickly recognize the risk that a fetus gets cerebral hypoxia.

SUMMARY OF THE INVENTION

It is therefore an object of the present invention to provide a fetal-pulse-wave-velocity-related-information obtaining apparatus and a childbirth monitoring apparatus having the function of obtaining pulse-wave-velocity-related information, each of which can be used to quickly recognize the risk that a fetus gets cerebral hypoxia.

The above object has been achieved by the present invention according to which change of blood pressure of a fetus is monitored by iteratively obtaining respective sets of pulse-wave-velocity-related information that change in relation with the blood pressure.

The above object has been achieved by the present invention. According to a first aspect of the present invention, there is provided a fetal-pulse-wave-velocity-related-information obtaining apparatus, comprising a heartbeat-synchronous-signal detecting device at least a portion of which is adapted to be worn on at least one of a fetus and a mother so as to detect a heartbeat-synchronous signal produced from a proximal portion of the fetus; a pulse-wave detecting device which detects a pulse wave from a distal portion of the fetus that is located on a distal side of the proximal portion of the fetus; and a pulse-wave-velocity-related-information obtaining means for iteratively obtaining, based on the heartbeat-synchronous signal detected by the heartbeat-synchronous-signal detecting device and the pulse wave detected by the pulse-wave detecting device, respective sets of pulse-wave-velocity-related information each of which is related to a velocity at which the pulse wave propagates in the fetus.

According to this aspect, the pulse-wave-velocity-related-information obtaining means iteratively obtains the respective sets of pulse-wave-velocity-related information from the fetus. Thus, it is possible to monitor the change of blood pressure of the fetus by monitoring the change of the sets of pulse-wave-velocity-related information. Therefore, it is possible to quickly recognize a risk that the fetus gets cerebral hypoxia.

According to a second aspect of the present invention, there is provided a childbirth monitoring apparatus, comprising a labor-pain-waveform detecting device which detects a labor-pain waveform representing a change of a degree of a labor pain of a mother; a heart-rate-related-information obtaining device which iteratively obtains respective sets of heart-rate-related information each of which is related to a heart rate of a fetus; a heartbeat-synchronous-signal detecting device at least a portion of which is adapted to be worn on at least one of the fetus and the mother so as to detect a heartbeat-synchronous signal produced from a proximal portion of the fetus; a pulse-wave detecting device which detects a pulse wave from a distal portion of the fetus that is located on a distal side of the proximal portion of the fetus; and a pulse-wave-velocity-related-information obtaining means for iteratively obtaining, based on the heartbeat-synchronous signal detected by the heartbeat-synchronous-signal detecting device and the pulse wave detected by the pulse-wave detecting device, respective sets of pulse-wave-velocity-related information each of which is related to a velocity at which the pulse wave propagates in the fetus.

The above-described fetal-pulse-wave-velocity-related-information obtaining apparatus may be employed as part of the present childbirth monitoring apparatus. The present apparatus obtains the labor-pain waveform and iteratively obtains the sets of heart-rate-related information and the sets of pulse-wave-velocity-related information of the fetus. Therefore, a living person such as a medical person can quickly recognize a risk that the fetus gets cerebral hypoxia, by monitoring respective changes of the labor-pain waveform and the sets of heart-rate-related information, and the sets of pulse-wave-velocity-related information, of the fetus.

Here, preferably, the childbirth monitoring further comprising a display device; and a display control means for controlling the display device to display, along a common time axis, respective graphical representations of the labor-pain waveform continuously detected by the labor-pain-waveform detecting device, the respective sets of heart-rate-related information iteratively obtained by the heart-rate-related-information obtaining device, and the respective sets of pulse-wave-velocity-related information iteratively obtained by the pulse-wave-velocity-related- information obtaining means.

According to this feature, the display device can display, along a common time axis, the respective graphical representations of the labor-pain waveform, the heart-rate-related information, and the pulse-wave-velocity-related information. Therefore, a person can easily recognize respective degrees of change of the labor-pain waveform, the heart-rate-related information, and the pulse-wave-velocity-related information, and accordingly can more accurately recognize the risk that the fetus gets cerebral hypoxia.

BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:

Fig. 1 is a diagrammatic view for explaining a construction of a childbirth monitoring apparatus to which the present invention is applied;
Fig. 2 is a view for showing details of a construction of a labor-pain-signal sensor shown in Fig. 1;
Fig. 3 is a block diagram for explaining essential control functions of a CPU (central processing unit) employed in the monitoring apparatus of Fig. 1;
Fig. 4 is a flow chart for explaining the essential control functions of the CPU, shown in Fig. 1; and
Fig. 5 is a view for showing an example of graphs displayed by each of a display and a printer employed in the monitoring apparatus of Fig. 1.

DETAILED DESCRIPTION OF PREFERRED EMBODIMENTS

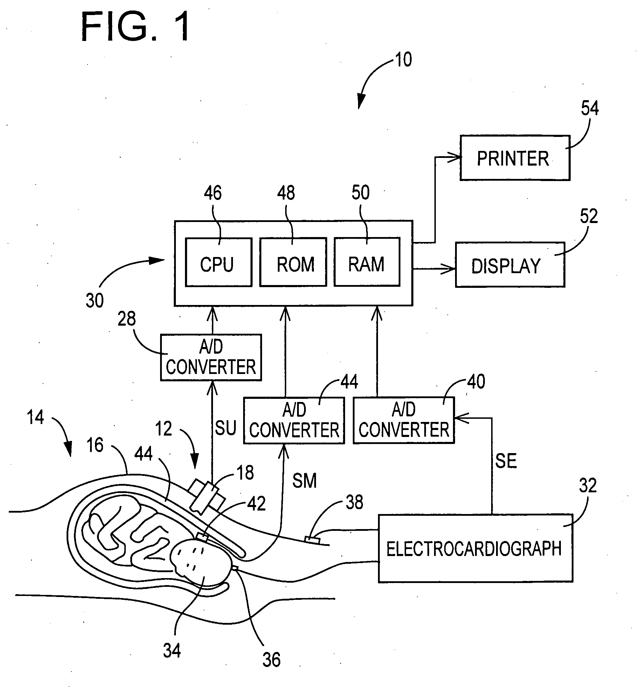

Hereinafter, there will be described an embodiment of the present invention in detail by reference to the accompanying drawings. Fig. 1 shows a diagrammatic view for explaining a construction of a childbirth monitoring apparatus 10 to which the present invention is applied. The present monitoring apparatus 10 also functions as a fetal-pulse-wave-velocity-related-information obtaining apparatus.

In Fig. 1, the present monitoring apparatus includes a labor-pain-signal sensor 12 which has a construction identical with that of a known external-type tocodynamometer and which is adapted to be fixed to an abdomen 16 of a mother 14 with the help of a fastening band 18.

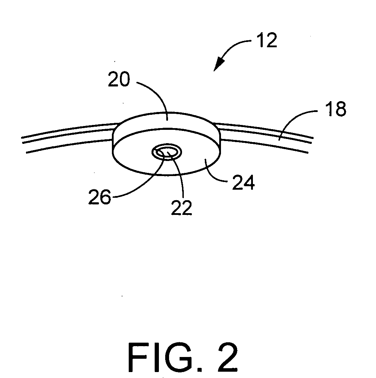

The construction of the labor-pain-signal sensor 12 is shown in detail in Fig. 2. More specifically described, the labor-pain-signal sensor 12 includes a disc-like guard ring 20 having an accommodating hole 26 formed in a central portion of a contact surface 24 thereof (i.e., a surface thereof opposed to the abdomen 16), and a pressure receiving element 22 that has a diameter slightly smaller than that of the accommodating hole 26 and is fitted in the hole 26. In a state in which the sensor 12 is worn on the abdomen 16 of the mother 14, the pressure receiving element 22 can detect a pressure caused by the contraction and hardening of uterus 44 of the mother.

Back to Fig. 1, the labor-pain-signal sensor 12 produces a labor-pain signal, SU, representing the thus detected pressure, and supplies the labor-pain signal SU to an electronic control device 30 via an A/D (analog-to-digital) converter 28. Since the labor-pain signal SU provides a labor-pain waveform, the labor-pain-signal sensor 12 functions as a labor-pain-waveform detecting device.

An electrocardiograph 32 incorporates a power supply and an amplifier (both not shown), and includes a first electrode 36 which is worn on a head of a fetus 34 after amniorrhexis and a second electrode 38 which is worn on a prescribed (e.g., femoral) portion of the mother 14 and cooperates with the first electrode 36 to continuously produce an electrocardiogram signal, SE. The electrocardiogram signal SE is amplified by the amplifier and the amplified signal SE is supplied to the control device 30 via an A/D converter 40. The electrocardiogram signal SE provides an electrocardiogram representing the action potential of cardiac muscle of the fetus 34. Since the electrocardiogram is a heartbeat-synchronous signal that is produced in synchronism with heartbeats of the fetus 34, the electrocardiograph 32 functions as a heartbeat-synchronous-signal detecting device. The first electrode 36 is an electrode which has a spiral needle at a tip thereof and is screwed into the skin of head of the fetus 34.

A photoelectric-pulse-wave sensor 42 is a sensor that detects a volumetric pulse wave (i.e., a plethysmographic signal) from peripheral blood vessels of a fetus, and is interposed between the cheek of the fetus 34 and the uterus 44 of the mother. The photoelectric-pulse-wave sensor 42 is of a reflection type, and includes a light emitter and a light receiver, both not shown. The light emitter is provided by, e.g., a light emitting diode, and emits, toward the skin of cheek of the fetus 34, a red or infrared light having a wavelength that can be reflected by hemoglobin, preferably a light having, e.g., a 800 nm wavelength that is not influenced by blood oxygen saturation. When the light emitted is incident to the skin of the fetus 34, a portion of the light is scattered by the red cells present in the skin or the subcutaneous tissue under the skin, and the scattered light is detected by the light receiving element. Thus, the scattered light represents a volumetric pulse wave produced from peripheral blood vessels of the fetus 34. The light receiving element produces a volumetric-pulse-wave signal, SM, representing the detected light, and the volumetric-pulse-wave signal SM is supplied to the control device 30 via an A/D converter 44. The volumetric pulse wave detected by the photoelectric-pulse-wave sensor 42 is a pulse wave that is produced from a distal portion of the fetus 34 that is located on a distal side of a proximal portion of the same 34 from which the electrocardiogram is produced as the electrocarodigram signal SE. Thus, the photoelectric-pulse-wave sensor 42 functions as a pulse-wave detecting device.

The electronic control device 30 is provided by a so-called microcomputer including a CPU (central processing unit) 46, a ROM (read only memory) 48, a RAM (random access memory) 50 and an I/O (input-and-output) port, not shown. The CPU 46 processes signals according to the control programs pre-stored in the ROM 48 by utilizing the temporary-storage function of the RAM 50, and iteratively determines a heart rate HR and a pulse-wave propagation time DT of the fetus 34. In addition, the CPU 46 operates a display 52 and a printer 54 each as a display device to iteratively display the respective determined values of heart rate HR and pulse-wave propagation time DT and continuously display the labor-pain waveform detected by the labor-pain-signal sensor 12. The display 52 may include a cathode ray tube (CRT).

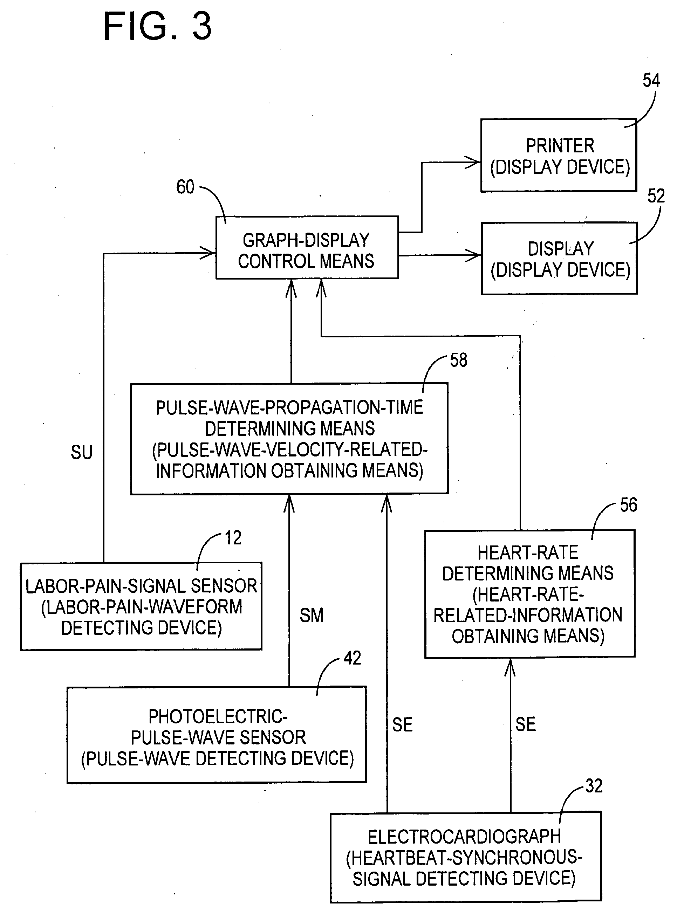

Fig. 3 is a diagrammatic view for explaining essential control functions of the CPU 46 of the present monitoring apparatus. A heart-rate determining device or means 56 as a sort of heart-rate-related-information obtaining means iteratively determines a heart rate HR of the fetus 34, based on the electrocardiogram signal SE continuously supplied from the electrocardiograph 32. In the present embodiment, the electrocardiograph 32 and the heart-rate determining means 56 cooperate with each other to provide a heart-rate-related-information obtaining device.

A pulse-wave-propagation-time determining device or means 58 as a sort of pulse-wave-velocity-related-information obtaining means determines, as a pulse-wave propagation time, DT (sec), a time difference between a time of detection of a prescribed periodic point, such as an R-wave, occurring to the electrocardiogram represented by the electrocardiogram signal SE continuously detected by the electrocardiograph 32, and a time of detection of a prescribed periodic point, such as a rising point or a peak point, occurring to the volumetric pulse wave continuously detected by the photoelectric-pulse-wave sensor 42. Pulse-wave-velocity-related information such as pulse-wave propagation time DT is a mathematical function of blood pressure or degree of arteriosclerosis. Since, however, arterial wall of the fetus 34 is soft, the pulse-wave-velocity-related information can be used as a parameter indicating blood pressure of the fetus.

A graph-display control device or means 60 operates each of the display 52 and the printer 54 to iteratively display, along a common time axis, the respective values of heart rate HR that are iteratively determined by the heart-rate determining means 56, and the respective values of pulse-wave propagation time DT that are iteratively determined by the pulse-wave-propagation-time determining means 58, and continuously display the labor-pain waveform that is continuously detected by the labor-pain-signal sensor 12.

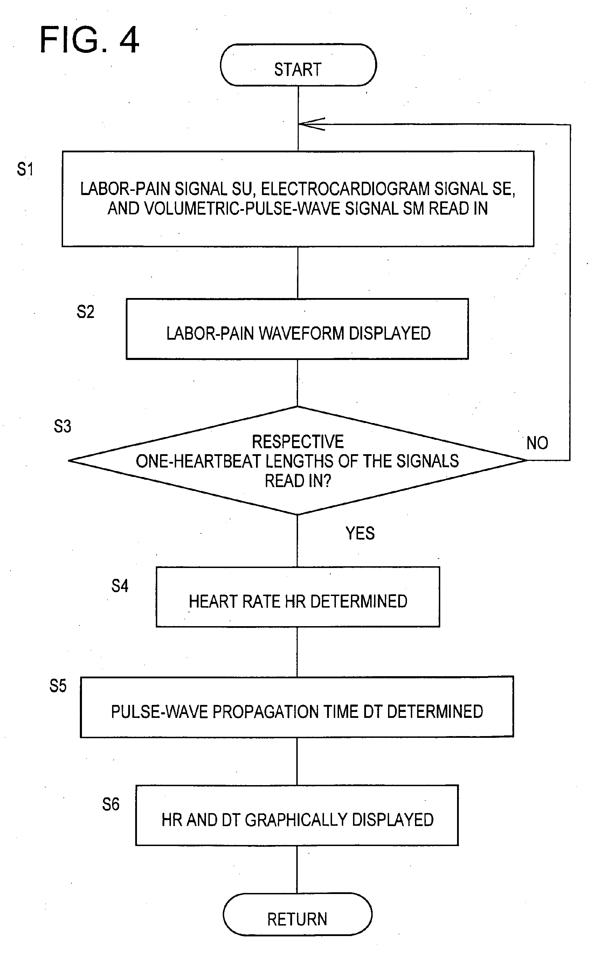

Fig. 4 is a flow chart for explaining the essential control functions of the CPU 46, shown in Fig. 3. First, at Step S1 (hereinafter, "Step" is omitted), the CPU reads in the labor-pain signal SU supplied from the labor-pain-signal sensor 12, the electrocardiogram signal SE supplied from the electrocardiograph 32, and the volumetric-pulse-wave signal SM supplied from the photoelectric-pulse-wave sensor 42.

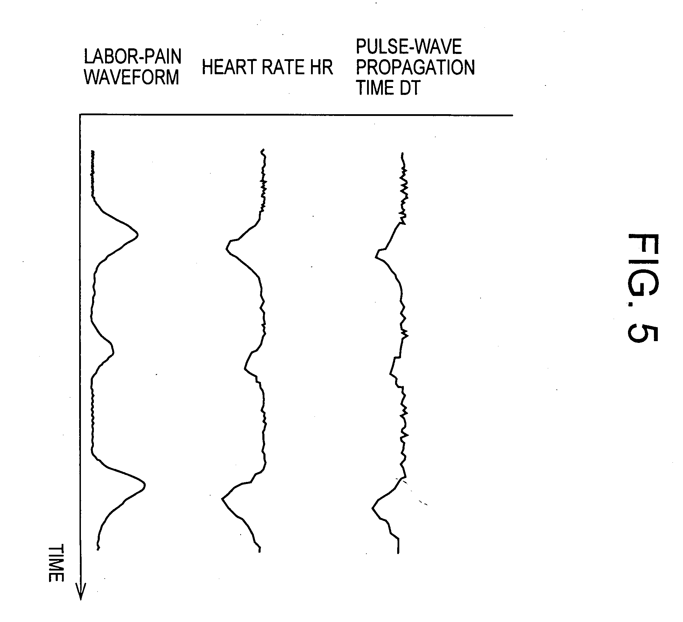

Subsequently, at S2, the CPU operates each of the display 52 and the printer 54 to display, as shown in Fig. 5, a labor-pain waveform represented by the labor-pain signal SU read in at S1, together with a time axis.

Then, at S3, the CPU judges whether the CPU has read in respective one-heartbeat lengths of the electrocardiogram signal SE and the volumetric-pulse-wave signal SM since a positive judgment was made at this step in the last control cycle in accordance with this routine. More specifically described, at this step, the CPU judges whether a prescribed periodic point has occurred to the electrocardiogram represented by the electrocardiogram signal SE, or whether a prescribed periodic point has occurred to the volumetric pulse wave continuously represented by the volumetric-pulse-wave signal SM. For example, the CPU judges whether a rising point (e.g., a minimum point) has occurred to the volumetric pulse wave.

If a negative judgment is made at S3, the control goes back to S1 and the following steps to continue reading in the signals. Meanwhile, if a positive judgment is made at S3, the control goes to S4 corresponding to the heart-rate determining means 56. At S4, the CPU determines, as a pulse period, RR (sec), a time interval between respective R-waves of successive heartbeat-synchronous pulses of the electrocardiogram that has been read in while S1 through S3 are repeated, and calculates a heart rate HR (times/min) based on the thus determined pulse period RR according to the following relationship: HR = 60/RR.

Then, the control goes to S5 corresponding to the pulse-wave-propagation-time determining means 58. At S5, the CPU determines, as a pulse-wave propagation time DT, a time difference between a time of occurrence of an R-wave of a heartbeat-synchronous pulse of the electrocardiogram that has been read in while S1 through S3 are repeated, and a time of occurrence of a rising point of a corresponding heartbeat-synchronous pulse of the volumetric pulse wave that has been read in while S1 through S3 are repeated.

Subsequently, at S6, the CPU operates each of the display 52 and the printer 54 to graphically display, along the time axis displayed at S2, the heart rate HR and the pulse-wave propagation time DT determined at S4 and S5, respectively. In the embodiment shown in Fig. 4, S2 and S6 correspond to the graph-display control means 60.

After S6, the control goes back to S1 and the following steps. Therefore, each of the display 52 and the printer 54 displays, along the common time axis, respective time-wise changes of the labor-pain waveform, the heart rate HR, and the pulse-wave propagation time DT corresponding to the blood pressure of the fetus 34, as shown in Fig. 5.

It emerges from the foregoing description of the childbirth monitoring apparatus 10 that the pulse-wave-propagation-time determining means 58 iteratively determines respective values of the pulse-wave propagation time DT of the fetus 34. Thus, a living person such as a doctor can monitor the blood pressure of the fetus 34 by monitoring the time-wise change of the pulse-wave propagation time DT. Therefore, the person can quickly recognize a risk that the fetus 34 may get encephalopathy.

In addition, the childbirth monitoring apparatus 10 continuously obtains the labor-pain waveform of the mother and iteratively obtains the respective values of the heart rate HR and the pulse-wave propagation time DT of the fetus 34. Thus, the person can quickly recognize a risk that the fetus 34 may get encephalopathy, by monitoring the respective time-wise changes of the labor-pain waveform of the mother, and the heart rate HR and the pulse-wave propagation time DT of the fetus 34.

In addition, in the childbirth monitoring apparatus 10, each of the display 52 and the printer 54 graphically displays, along the common time axis, the respective time-wise changes of the labor-pain waveform, the heart rate HR, and the pulse-wave propagation time DT. Thus, the person can easily recognize a degree of change of each of the labor-pain waveform, the heart rate HR, and the pulse-wave propagation time DT. Therefore, the person can more accurately recognize a risk that the fetus 34 may get encephalopathy.

While the present invention has been described in its preferred embodiment by reference to the drawings, it is to be understood that the invention may otherwise be embodied.

For example, in the illustrated embodiment, the labor-pain-signal sensor 12 is of the external type in which the sensor 12 is worn on the abdomen of the mother 14 so as to detect the labor-pain signal SU. However, the external-type sensor 12 may be replaced with an internal-type sensor that measures an amniotic pressure or a pressure between fetus and parturient canal and thereby produces a labor-pain signal SU.

Also, in the childbirth monitoring apparatus 10, the first and second electrodes 36, 38 are employed so as to obtain the electrocardiogram from the fetus, and are used such that the first electrode 36 is worn on the head of the fetus 34 and the second electrode 38 is worn on the femoral portion of the mother 14. However, the total number of the electrodes employed or the places where the electrodes are worn are not limited to the details of the illustrated embodiment. For example, both of the two electrodes may be worn on the mother 14; or a third and a fourth electrode may be additionally employed so as to subtract an electrocardiogram signal produced from the mother.

Also, in the childbirth monitoring apparatus 10, the heart-rate determining means 56 determines the heart rate HR as a sort of heart-rate-related information. However, the heart rate HR may be replaced with the pulse period RR.

Also, in the childbirth monitoring apparatus 10, the heart rate HR is determined based on the electrocardiogram signal SE. However, a heart rate HR may be determined based on the volumetric pulse wave detected by the photoelectric-pulse-wave sensor 42. Otherwise, a microphone may be worn on the skin of the abdomen 16 of the mother 14 so as to detect heart sounds of the fetus 34, and a heart rate HR may be determined based on the heart sounds detected by the microphone.

Also, in the childbirth monitoring apparatus 10, the electrocardiograph 32 that detects the electrocardiogram signal SE is employed as a sort of heartbeat-synchronous-signal detecting device. However, the heartbeat-synchronous-signal detecting device may be provided by a microphone which is worn on the skin of the abdomen 16 of the mother 14 so as to detect heart sounds of the fetus 34.

Also, in the childbirth monitoring apparatus 10, the photoelectric-pulse-wave sensor 42 is interposed between the cheek of the fetus 34 and the uterus 44. However, the sensor 42 may be worn directly on the fetus 34.

Also, in the childbirth monitoring apparatus 10, the pulse-wave propagation time DT is obtained as a sort of pulse-wave-velocity-related information. However, a pulse-wave velocity PWV that is calculated based on the pulse-wave propagation time DT may be obtained as the pulse-wave-velocity-related information. The pulse-wave velocity PWV may be calculated based on the pulse-wave propagation time DT according to the following Expression 1:

$$(\text{Expression 1}) \qquad PWV = L/DT$$

where L is a distance between the portion where the heartbeat-synchronous signal is produced and the portion where the pulse wave is produced, and is replaced with a prescribed constant.

It is to be understood that the present invention may be embodied with other changes, improvements, and modifications that may occur to a person skilled in the art without departing from the spirit and scope of the invention defined in the appended claims.

**Claims**

1. A fetal-pulse-wave-velocity-related-information obtaining apparatus (10), comprising:

   a heartbeat-synchronous-signal detecting device (32) at least a portion of which is adapted to be worn on at least one of a fetus and a mother so as to detect a heartbeat-synchronous signal produced from a proximal portion of the fetus;
   a pulse-wave detecting device (42) which detects a pulse wave from a distal portion of the fetus that is located on a distal side of the proximal portion of the fetus; and
   a pulse-wave-velocity-related-information obtaining means (58) for iteratively obtaining, based on the heartbeat-synchronous signal detected by the heartbeat-synchronous-signal detecting device and the pulse wave detected by the pulse-wave detecting device, respective sets of pulse-wave-velocity-related information each of which is related to a velocity at which the pulse wave propagates in the fetus.

2. A childbirth monitoring apparatus, comprising:

   a labor-pain-waveform detecting device (12) which detects a labor-pain waveform representing a change of a degree of a labor pain of a mother;
   a heart-rate-related-information obtaining device (32, 56) which iteratively obtains respective sets of heart-rate-related information each of which is related to a heart rate of a fetus;
   a heartbeat-synchronous-signal detecting device (32) at least a portion of which is adapted to be worn on at least one of the fetus and the mother so as to detect a heartbeat-synchronous signal produced from a proximal portion of the fetus;
   a pulse-wave detecting device (42) which detects a pulse wave from a distal portion of the fetus that is located on a distal side of the proximal portion of the fetus; and
   a pulse-wave-velocity-related-information obtaining means (58) for iteratively obtaining, based on the heartbeat-synchronous signal detected by the heartbeat-synchronous-signal detecting device and the pulse wave detected by the pulse-wave detecting device, pulse-wave-velocity-related information that is related to a velocity at which the pulse wave propagates in the fetus.

3. A childbirth monitoring apparatus according to claim 2, further comprising
   a display device (52, 54); and
   a display control means (60) for controlling the display device to display, along a common time axis, respective graphical representations of the labor-pain waveform continuously detected by the labor-pain-waveform detecting device, the respective sets of heart-rate-related information iteratively obtained by the heart-rate-related-information obtaining device, and the respective sets of pulse-wave-velocity-related information iteratively obtained by the pulse-wave-velocity-related-information obtaining means.

4. A fetal-pulse-wave-velocity-related-information obtaining apparatus according to any of claims 1 through 3, wherein the heartbeat-synchronous-signal detecting device comprises an electrocardiograph (32) which includes at least one electrode (36, 38) which is adapted to be worn on at least one of the fetus and the mother and which detect, as the heartbeat-synchronous signal, an electrocardiogram including a plurality of heartbeat-synchronous pulses produced in synchronism with respective beats of a heart of the fetus, wherein the pulse-wave detecting device comprises a volumetric-pulse-wave sensor (42) which detects, as the pulse wave, a volumetric pulse wave from a peripheral portion of the fetus, the volumetric pulse wave including a plurality of heartbeat-synchronous pulses produced in synchronism with the respective beats of the heart of the fetus, and wherein the pulse-wave-velocity-related-information obtaining means (58) iteratively obtains, based on each one of said plurality of heartbeat-synchronous pulses of the electrocardiogram and a corresponding one of said plurality of heartbeat-synchronous pulses of the volumetric pulse wave, a set of pulse-wave-velocity-related information which is related to a velocity at which said one heartbeat-synchronous pulse of the volumetric pulse wave propagates from the heart, to the peripheral portion, of the fetus.

5. A fetal-pulse-wave-velocity-related-information obtaining apparatus according to claim 4, wherein the pulse-wave-velocity-related-information obtaining means comprises a pulse-wave-propagation-time determining means (58) for determining a time difference between a time of detection of a prescribed point of said each one of said plurality of heartbeat-synchronous pulses of the electrocardiogram and a time of detection of a prescribed point of said corresponding one of said plurality of heartbeat-synchronous pulses of the volumetric pulse wave, as a pulse-wave propagation time as said set of pulse-wave-velocity-related information.

# FIG. 1

FIG. 2

# FIG. 3

```
                                                    ┌──────────────────┐ ⌐54
                                                    │     PRINTER      │
                                                    │ (DISPLAY DEVICE) │
                                                    └──────────────────┘
                    ⌐60                                                    ⌐52
        ┌─────────────────┐                         ┌──────────────────┐
        │  GRAPH-DISPLAY  │──────────────────────── │     DISPLAY      │
   ────▶│ CONTROL MEANS   │───────────────────────▶ │ (DISPLAY DEVICE) │
        └─────────────────┘                         └──────────────────┘
```

GRAPH-DISPLAY CONTROL MEANS — 60

PRINTER (DISPLAY DEVICE) — 54

DISPLAY (DISPLAY DEVICE) — 52

SU

PULSE-WAVE-PROPAGATION-TIME DETERMINING MEANS (PULSE-WAVE-VELOCITY-RELATED-INFORMATION OBTAINING MEANS) — 58

LABOR-PAIN-SIGNAL SENSOR (LABOR-PAIN-WAVEFORM DETECTING DEVICE) — 12

SM

HEART-RATE DETERMINING MEANS (HEART-RATE-RELATED-INFORMATION OBTAINING MEANS) — 56

PHOTOELECTRIC-PULSE-WAVE SENSOR (PULSE-WAVE DETECTING DEVICE) — 42

SE

SE

ELECTROCARDIOGRAPH (HEARTBEAT-SYNCHRONOUS-SIGNAL DETECTING DEVICE) — 32

10

# FIG. 4

START

**S1**
LABOR-PAIN SIGNAL SU, ELECTROCARDIOGRAM SIGNAL SE, AND VOLUMETRIC-PULSE-WAVE SIGNAL SM READ IN

**S2**
LABOR-PAIN WAVEFORM DISPLAYED

**S3**
RESPECTIVE ONE-HEARTBEAT LENGTHS OF THE SIGNALS READ IN?

NO

YES

**S4**
HEART RATE HR DETERMINED

**S5**
PULSE-WAVE PROPAGATION TIME DT DETERMINED

**S6**
HR AND DT GRAPHICALLY DISPLAYED

RETURN

# FIG. 5

**EP 1 366 709 A1**

European Patent
Office

**EUROPEAN SEARCH REPORT**

Application Number

EP 03 00 6242

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | US 5 419 322 A (GUZMAN FRANCINE A ET AL) 30 May 1995 (1995-05-30) * abstract * | 1-5 | A61B5/0285 |
| Y | US 6 331 162 B1 (MITCHELL GARY F) 18 December 2001 (2001-12-18) * abstract * * figure 2 * | 1-5 | |
| Y | US 5 184 619 A (AUSTIN SANDOR) 9 February 1993 (1993-02-09) * abstract * * figure 1 * | 2,3 | |
| A | US 4 660 564 A (GENNSER GERHARD M ET AL) 28 April 1987 (1987-04-28) * column 3, paragraph 4 * | 1-5 | |
| A | GARDINER H ET AL: "Ventriculovascular physiology of the growth-restricted fetus." ULTRASOUND IN OBSTETRICS & GYNECOLOGY: THE OFFICIAL JOURNAL OF THE INTERNATIONAL SOCIETY OF ULTRASOUND IN OBSTETRICS AND GYNECOLOGY. ENGLAND JUL 2001, vol. 18, no. 1, July 2001 (2001-07), pages 47-53, XP002254988 ISSN: 0960-7692 * Abstract * | 1-5 | **TECHNICAL FIELDS SEARCHED (Int.Cl.7)** A61B |

The present search report has been drawn up for all claims

| Place of search | Date of completion of the search | Examiner |
|---|---|---|
| MUNICH | 19 September 2003 | Edward, V |

## ANNEX TO THE EUROPEAN SEARCH REPORT
## ON EUROPEAN PATENT APPLICATION NO.

EP 03 00 6242

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2003

| Patent document cited in search report | | Publication date | Patent family member(s) | | Publication date |
|---|---|---|---|---|---|
| US 5419322 | A | 30-05-1995 | NONE | | |
| US 6331162 | B1 | 18-12-2001 | NONE | | |
| US 5184619 | A | 09-02-1993 | NONE | | |
| US 4660564 | A | 28-04-1987 | DE | 3512053 A1 | 31-10-1985 |
| | | | FR | 2563991 A1 | 15-11-1985 |
| | | | GB | 2156985 A ,B | 16-10-1985 |
| | | | JP | 1656191 C | 13-04-1992 |
| | | | JP | 3022779 B | 27-03-1991 |
| | | | JP | 60259250 A | 21-12-1985 |
| | | | SE | 456482 B | 10-10-1988 |
| | | | SE | 8500608 A | 03-10-1985 |

EPO FORM P0459

For more details about this annex : see Official Journal of the European Patent Office, No. 12/82